Europäisches Patentamt

European Patent Office

Office européen des brevets

(19)

(11) Publication number: **0 393 289**

**A1**

## (12) EUROPEAN PATENT APPLICATION

(21) Application number: **89402429.8**

(22) Date of filing: **06.09.89**

(51) Int. Cl.⁵: **A61K 7/00**

(30) Priority: **19.04.89 US 340258**

(43) Date of publication of application:
**24.10.90 Bulletin 90/43**

(84) Designated Contracting States:
**BE DE FR GB IT NL**

(71) Applicant: **Fellows, Charles Thomas**
**239 East Helena Street**
**Dayton Ohio 45404(US)**

Applicant: **Berman, Michael**
**350 Fifth Avenue**
**New-York New York 10021(US)**

(72) Inventor: **Fellows, Charles Thomas**
**239 East Helena Street**
**Dayton Ohio 45404(US)**
Inventor: **Berman, Michael**
**350 Fifth Avenue**
**New-York New York 10021(US)**

(74) Representative: **Peaucelle, Chantal et al**
**S.C. Ernest Gutmann - Yves Plasseraud 67,**
**boulevard Haussmann**
**F-75008 Paris(FR)**

(54) **Microencapsulated particulates in dry powder form, products containing same and methods of making same.**

(57) A method produces a water-insoluble liquid microencapsulated particulate in dry powder form. A precondensate mixture is reacted and polymerized under suitable conditions, thereby producing the encapsulated particulate. A product also is made from the encapsulated particulates substantially non-adhered to a carrier substrate. The method of making the product comprises preparing a slurry by contacting the encapsulated particulates with a suspending agent and with a wetting agent and applying the slurry to the carrier substrate, and evaporating the wetting agent from the applied slurry, thereby preparing the microencapsulated liquid product.

# MICROENCAPSULATED PARTICULATES IN DRY POWDER FORM, PRODUCTS CONTAINING SAME AND METHODS OF MAKING SAME

## BACKGROUND OF THE INVENTION

Printed materials which contain microencapsulated fragrances are used by the fragrance industry for promotional advertising and sampling fragrance products. Two examples of these commercial materials are the pull-apart scent strip and the scratch-and-sniff scent strip, both of which have microencapsulated fragrances deposited thereon and held in place with adhesives.

The microencapsulated scratch-and-sniff scent strips are manufactured by converting essential fragrance oils, i.e. "perfumes", into an encapsulated form which is mixable with water. The encapsulated fragrance and water combination is formulated into a printable or coatable ink which contains water soluble binders, such as water soluble polymers. This ink solution is then applied as a film to a paper surface. When dry, the water soluble polymers, which act as adhesives, hold the encapsulated fragrances in place on the paper sheet until the capsules are to be ruptured under pressure, causing the fragrance to be released. Since long prior to 1986, methods of this type generally have been suggested for use by the cosmetic industry to supply a product for sampling cosmetics (see U.S. Patent No. 4,752,496).

The pull-apart scent strip fragrance sampler (see U.S. Patent No. 4,720,417) is of similar construction in that the microencapsulated slurry is applied to a substrate. However, in the manufacture of that product, the adhesive and encapsulated fragrance mixture acts as a dry mastic which adheres two opposing paper surfaces, bonding them temporarily together. The cohesive strength of the connecting composition or bonding coating is very poor, so that when the two opposing paper surfaces are pulled apart, the capsules containing the fragrance are ruptured and the fragrance is released. Products of this general type also have been available since long prior to 1986.

The pull-apart scent strip requires a very distinct and exacting co-acting relationship in physical properties between the paper carrier, the adhesive, the encapsulating material containing the product, the volume of the microcapsules contained in the adhesive, the adhesive and cohesive strength of the adhesive containing the microcapsules and the force required to separate the temporarily bonded sheets of paper to allow the microcapsules to release their contents.

Prior to deposition on either the pull-apart or scratch-and-sniff scent strips, the fragrance must be converted into a microencapsulated form. There are many methods available to encapsulate or form a shell around a particle, such as a fragrance or oil droplet. One method of forming microencapsulates utilizes a gelatin/gum arabic/polyvinyl methyl ether-maleic anhydride solution. (see British Specification No. 1,129,430). Another technique is aldehyde condensation polymerization. Aldehyde condensation is a desirable reaction method for encapsulating oil droplets because control of the raw materials, such materials ordinarily being readily synthesized, is relatively easily maintained. Moreover, the polymer precursors are usually completely soluble in aqueous solution and become insoluble upon further condensation polymerization, whereupon they readily precipitate out of the aqueous phase.

Aminoplast polymers are especially useful as a reactant in the aldehyde condensation reaction. Aminoplast polymers, and particularly melamine/urea formaldehyde plastics, may be used to encapsulate liquid materials in the polymerization process. These polymers produce impressive results in the capsule size range of 50 microns and less, e.g. about 5 to 25 microns, more or less. Such capsules have been used extensively in self-contained impact papers since about 1963. In such use and combination, they have demonstrated toughness, impermeability and general superiority. These capsules also have exhibited sufficient toughness and impermeability to carry non-aqueous solutions without premature rupture or leakage. The capsules can be stored or used under normal handling conditions for months and even years without premature capsule rupture. Further, capsules made of aminoplast polymers can stand the rigors of further formulation into secondary systems, such as printing inks or coatings for use in printing equipment, and can therefore be directly incorporated into a final paper product. This property allows the use of printing techniques to deposit the capsules onto the paper wherein the capsules are adhered or at least partially adhered to the paper surface in the final product. The liquid filled capsules remain in the proximity of the paper surface until the desired time when the capsules are ruptured to release the capsule contents.

## SUMMARY OF THE INVENTION

It is an object of this invention to provide methods for encapsulating water-insoluble liquids, such as perfumes, in dry powder form.

It is another object of this invention to provide microencapsulated liquids in dry powder form and products containing the encapsulated liquids.

It is still another object of this invention to provide suitable methods for making products containing microencapsulated particles in dry powder form.

To these and other ends, this invention provides a method of producing a water-insoluble liquid microencapsulated particulate. The method comprises reacting a precondensate mixture and polymerizing the precondensate mixture so formed under suitable conditions, thereby producing an encapsulated particulate. The invention also provides a product which comprises the encapsulated particulates so formed and a carrier substrate. The method of the invention further comprises preparing a slurry by contacting the encapsulated particulates with a suspending agent and a wetting agent, under suitable conditions, and applying the slurry so formed to the carrier substrate, evaporating the wetting agent from the applied slurry, thereby preparing the microencapsulated liquid product.

## DETAILED DESCRIPTION OF THE INVENTION

Generally, the invention provides a product for dispensing a liquid in the form of a microencapsulated particulate from a carrier substrate, such as a paper base, wherein the encapsulated particulates are carried substantially non-adhered to the carrier substrate. The product is particularly useful for carrying encapsulated water-insoluble liquid materials, such as fragrances.

The invention also provides a method of encapsulating liquid material. The method comprises reacting a precondensate mixture under suitable conditions and polymerizing the precondensated mixture so formed, under suitable conditions, to form the encapsulated particulates.

More specifically, the method contemplates use of an aqueous solution of a water soluble, low molecular weight melamine/urea aldehyde precondensate. The precondensate predominantly comprises low molecular weight reaction products of melamine, urea and formaldehyde, e.g. dimethylol urea. The solution has a solid content of about 3% to about 80% by weight of the total aqueous precondensate. A crosslinking agent, such as poly vinyl methyl ether - maleic anhydride (PVE/MA), is used in a solids content of about 0.5% to about 20% by weight of the total aqueous precondensate. A water-insoluble liquid material is incorporated to form the capsule core in an amount in parts by weight of about one-tenth of a part to about ten parts of fill material for each part solids in the precondensate. The fill material is dispersed as microscopically sized discrete particles in the solution in the substantial absence of wetting agents, while continuing to maintain the fill material in particulate form and maintaining the resultant dispersion at a temperature of about $10°$ C to about $50°$ C.

Polymerization by acid catalysis of the precondensate is initiated by the addition of acid in an amount to provide a pH for the dispersion in the range of about 1.5 to about 5.0, and preferably from about 1.5 to about 4.5. The polymerization is continued while maintaining the liquid core material as finely dispersed particles, that is, by rapid agitation within a temperature range of about $15°$ C to about $50°$ C for at least one hour, or until an aqueous slurry of capsules is formed.

This slurry comprises particles of liquid core material encapsulated within tough, water insoluble, melamine/urea-formaldehyde PVE/MA polymer shells. The resulting microencapsuled particulate slurry then is neutralized and stored or used in slurry form. The capsules also may be separated by simple filtration, dried and used in dry, free-flowing form.

Precondensate reaction products which are useful in the practice of the invention are those which comprise predominantly reaction products of melamine/urea and formaldehyde with polyvinyl methyl ether-maleic anhydride, and which are capable of polymerization from a water soluble prepolymer state under acid conditions in aqueous media (i.e., at a pH less than 7) to substantially water insoluble polymers. The water soluble prepolymers are generally made by reacting melamine/urea and formaldehyde in an equivalent weight ratio of about 0.6 to about 1.3 parts of formaldehyde to about 1 part of melamine/urea, and preferably in a weight ratio of about 1 part of formaldehyde to about 1 part of melamine/urea. This is a mole ratio of about 1.2 to about 2.6 parts of formaldehyde to about one part of melamine/urea, and preferably a mole ratio of about 2:1. Thiourea, cyanuramide (melamine), guanidine, N-alkyl ureas, phenols, sulfonamides, anilines, amines and the like may be included as modifiers for the melamine/urea. When modifiers are used, they should replace no more than about 25% of the urea, preferably to replace no more than 10% of the urea, the percentage being calculated on an equivalent weight basis.

Suitable water soluble melamine/urea-aldehyde prepolymers for use in the practice of this invention are commercially available for use as adhesives, for example, Cymel 323, 325, 327, 373, 385 and Beetle 60 resins marketed by American Cyanamid (at about 80% solids in aqueous solution) and "Resimene" 714, 717, and 721 resins marketed by Monsanto (at about 80% solids in aqueous solution). The polyvinyl methyl ether-maleic anhydride resin is supplied in a 100% dry resin form marketed by GAF Chemicals.

It is also noted that superior capsules are made from melamine/urea-formaldehyde/PVE/MA prepolymers or precondensates prepared by the weakly acidic catalyzed reaction of melamine/urea and formaldehyde in water under carefully controlled conditions. Preferred conditions for the preparation of the precondensate solution are pH values in the range of from about 4.5 to about 5.0 and temperatures from about 15° C to about 25° C. The precondensate can be made at room temperature and held from 4 to 24 hours before use.

When the concentration of the precondensate at the time of acid addition is more than about 30 weight percent reactive solids, excessive viscosity may result, and if less than about 3 weight percent solids, the resulting capsules may be too weak and the economics of the encapsulation process decline. A preferable range of precondensate concentration in the aqueous medium, for producing superior capsules, is from about 10 to about 25 weight percent solids.

In general, the core material to be contained within the microcapsules can be liquid, solid or gaseous, so long as it is relatively insoluble (less than 5 or 10%) in the system and inert toward reaction with other ingredients in the system. In particular, the fill material should be inert to attack by the polymerization catalyzing acid. It is, however, in the area of liquid droplet filled capsules that the invention may have its greatest utility.

During encapsulation, the fill material must be maintained in the form of dispersed particles, usually as oily liquid droplets dispersed in the aqueous phase by vigorous agitation. In general, the more vigorous the agitation, the smaller the capsules produced. However, in practicing the method, there are other factors which bear on capsule size, such as the manner of precondensate preparation, acid addition rate and concentration, reaction temperature, and the type of fill used. The more readily dispersible the liquid non-water soluble material which forms the core contents of the capsule, the more readily that smaller capsules, i.e. below about 25 microns, are formed. Generally, water insoluble odoriferous liquids such a scents, essences and fragrances and the like are examples of useful liquid fills. Solid particles may be dissolved or dispersed in the liquid droplets. The liquids may also carry soluble dyes to impart certain desired color effects.

Preferably, the amount in parts by weight of the liquid core material based on the reactive precondensate solids, is about 0.7 to about 8 parts of liquid core material per one part of the precondensate solids. This range provides capsules containing from about 50% to about 80% core material and possessing sufficient toughness for normal handling in subsequent operations, such as formulation of printable inks or coatings for scented graphic demonstrations. For larger microcapsules or higher density cores or where more capsule rupture is desired and the amount of liquid core per capsule is not important, less than one-tenth part liquid core to each part condensate solids can be used to produce capsules.

After the fill material has been dispersed in the precondensate and the agitation rate properly adjusted, condensation polymerization of the melamine/urea-aldehyde/PVE/MA precondensate may be initiated by the addition of acid to the system. In general, any water soluble acid may be used, so long as it will maintain the pH in the range of about 1 to about 5. Examples of suitable acid catalysts include formic acid, citric acid, para toluene sulfonic acid, hydrochloric acid, sulfuric acid, phosphoric acid, and the like.

When capsule shell formation is begun by acid catalyst addition to the agitated dispersion of the liquid core in the precondensate solution, it is observed that the condensation polymerization initially proceeds slowly. Fill can be added after, instead of before, acid addition (but prior to shell formation). Shell walls are formed within the first hour after the pH is in the range of about 1 to about 5.

Variations of pH outside of this range result in capsules having generally undesirable properties. For calculating time of polymerization following acid addition, the polymerization time begins to run when the pH of the system reaches about 4.5. Thereafter, it may be adjusted further downward during the polymerization and such adjustment is part of the total polymerization time. A preferred pH range is from about 1.5 to about 3.5 to produce tougher, more impermeable capsules. Also, in this range, temperature control is easily maintained and capsule reproducibility is excellent. Capsules made at higher pH values than about 4.5 tend to be weak and too permeable for use without premature leakage of the liquid scented core. Below a pH of about 1.5, control of acidity is difficult and the equilibrium of the system may become such that shell wall formation is too rapid for adequate product control, and/or shell formation may be accompanied by polymer degradation.

The method of the invention produces capsules which have a diameter from about 5 microns to about 200 microns. When capsules of small sizes, e.g. 25 microns and smaller, are desired, the acid should be added to the system slowly over a period of several minutes, usually in increments over a period of about

4

10 minutes to an hour, and at a temperature in the range of about 20°C to about 30°C. In a three increment acid addition procedure, capsules of an average size of 5 to 20 microns result if the third increment is withheld for a period of from 30 to 60 minutes or more. When the reaction mixture containing the oily droplets is subjected to extended high speed agitation at this stage, smaller capsules result. If larger capsules are desired, the acid addition need not be so carefully controlled, and it may even be desirable to add the acid all at once.

After a polymerization period of about one-half hour to one hour following acid addition, the temperature may be raised to the range of 30°C to 45°C to complete the shell formation. The temperature, of course, should not be above the boiling point of either the liquid core material or the precondensate solution. Preferably, the reaction should be run for at least about 1 to 3 hours after acid addition at the desired pH range before separating the capsules or neutralizing the resultant slurry and preventing further significant polymerization. While the polymerization may be permitted to run as long as six hours or more, unless the fill is of a nature which prohibits too much temperature rise, e.g. above 40-45°C, no particular advantages are apparent. Tough, generally impermeable capsules are formed after one hour in the lower pH ranges. At higher temperatures, however, more consistent capsules are obtained if the polymerization is allowed to proceed for more than one hour.

Addition of the liquid core material may be made directly to the precondensate solution. However, since this precondensate solution usually is weakly acidic, it is preferred to adjust the initial pH of the precondensate solution to a lower acid state of about 4.0 before addition of the core material.

In the event the capsules are separated from the slurry, they can be dried by conventional drying procedures to a dry, free-flowing state. In practice, drying temperatures employed are in the range of about 60°F to about 350°F, that is, a temperature compatible with the fill material.

The determination of the percent of liquid core in any given capsule may be made by laboratory techniques. One method which is used is to crush a weighed sample of the capsules and thereafter extract the liquid core from the crushed samples. The weight loss, or the weight of the liquid obtained from the extraction solvent by evaporating the solvent, may be used as the basis for determining the relationship between core weight and shell wall weight. A second method is to hydrolyze the shells in concentrated hydrochloric acid and weigh the released liquid core following its separation from the aqueous acid.

Capsules of melamine/urea-formaldehyde/PVE/MA condensation polymer shells made in accordance with the practice of this invention are surprisingly tough and have been found sufficiently impermeable to retain volatile fragrances at elevated temperatures of 80°C for 72 hours and longer at atmospheric pressure with no measurable loss of fragrance strength or fidelity. The capsules display a rupture strength from about 5 g/cm to about 500 g/cm (at 20°C at 50% RH).

Capsule size may be determined by use of a "Coulter" counter, a particle size measuring unit marketed by Coulter Electonics, Inc. Size also may be determined microscopically either by the use of a microscope with an eyepiece micrometer calibrated to show actual parti cle size or by visual comparison of capsules with a size range of various standardized glass beads.

In general, the capsules of this invention display no special sensitivity to storage and can be stored for extended periods. Likewise, storage of aqueous slurries of capsules show no observable detrimental effect over long periods.

Dyes can be used to color the shell walls instead of being contained in the fill. To dye the normally transparent shell walls, the microcapsules are slurried in aqueous dye solution. For example, addition of Rhodamine B to the reaction mixture imparts a reddish-violet fluorescent color to the shell walls.

The following examples comprise reaction mixtures which may be used in accordance with the practice of this invention to prepare encapsulated particulates:

| Example A: | Material | Parts by Weight |
|---|---|---|

    1.    Water........................................50.85

    2.    Poly methyl vinyl ether/maleic
          anhydride........................... 1.75

    3    Melamine/urea-aldehyde
          precondensate.......................12.30

    4.    Liquid core phase, essential
          fragrance oil.......................35.10

**Example B:**

    1.    Water...............................33.56

    2.    Poly methyl vinyl ether/maleic
          anhydride..........................1.16

    3.    Melamine/urea-aldehyde
          precondensate......................8.12

    4.    Liquid core phase, essential
          fragrance oil......................57.16

**Example C:**

    1.    Water................................63.56

    2.    Poly methyl vinyl ether/maleic
          anhydride..........................2.19

    3.    Melamine/urea/aldehyde
          precondensate......................15.38

    4.    Liquid core phase, essential
          fragrance oil......................18.87

Processing Procedure:

    A. Add the PVE/MA to the water. Under agitation heat the material to 60°C and maintain until in solution. Remove from heat and cool to room temperature. At room temperature, adjust the pH to 4.5.

    B. Under agitation and at a temperature of 20°C add the melamine/urea-aldehyde precondensate to the PVE/MA solution.

    C. Continuing to agitate and at a temperature of 20°C, add the water insoluble essential fragrance oil to the above mixture and disperse to a micro droplet size of 10 to 15 microns.

D. When the desired micro droplet size is attained and while maintaining agitation, adjust the pH of the mixture to 4.0, using a 10% dilution of formic acid, and begin heating to 40°C.

E. At a temperature of 40°C maintain, agitating for 1 hour.

F. After maintaining constant agitation at 40°C for the 1 hour period, adjust the pH to 3.5 adding, dropwise, a 10% dilution of formic acid.

G. Allow the mixture to continue agitation for another 1 hour period at 40°C.

H. Remove heating and allow the formed capsules to come to room temperature.

I. Filter the capsules from the supernate and hold for further processing.

The encapsulated fragrances then must be provided in a suitable form for deposition onto the carrier substrate. The invention further provides a method for depositing the encapsulates onto the carrier substrate. The method comprises preparing a slurry which comprises about 20% to about 45% capsule solids by weight. The encapsulated particulates are carried in a volatile solvent and deposited on the carrier substrate using printing or coating techniques, in a substantially dry powder layer. A suspending agent such as polyethylene oxide or hydrophilic fumed silicate (for example, Cab-O-Sil marketed by the Cabot Corporation) is added at from about .05% to about 5% by weight to the slurry. Water may make up 100% of the volatile solvent. Isopropyl alcohol may be added to the water phase to accelerate the evaporation rate for high speed production rates. If desired, the isopropyl alcohol is added to the slurry at from about 5% to about 20% by weight of the weight of the water and more particularly at from about 5% to about 10% by weight.

Light tackifiers also may be used to temporarily hold, but not to adhere, the fragrance capsules in place on the substrate surface. Tackifiers such as water soluble polymers may be employed where the pigment-volume-concentration (PVC) between the weight of the fragrance capsules and the polymer solids has to be binder starved, i.e., less than the minimum amount of binder present, to aggressively bind the fragrance capsules to the receiving paper surface. The ratio of fragrance capsule solids to adhering polymer solids in the slurry is from about 100:1 to about 20:1. Suitable water soluble polymers which may be used are polyvinyl alcohol, polyvinyl pyrrolidone, hydroxy-ethyl cellulose, acrylic acids and natural gums such as gum arabic.

In accordance with the practice of the invention, the printed, coated or otherwise applied fragrance capsules and aqueous carrier is dried to remove the water solvent, leaving the microcapsules of fragrance deposited in a non-adhered layer upon one surface of the substrate. Once dried, the fragrance capsules have relatively little or no permanent adhesion to the surface. When in such a non-adhered state, the capsules can be dislodged or removed from the carrier and may be ruptured to release the fragrance for examination and use.

Deposition of the encapsulated fragrance slurry should be at a thickness of from about 0.01 millimeters to about 10.0 millimeters and preferably from about 1.0 millimeters to about 3.0 millimeters in thickness.

The volatile carrier can be removed under ambient temperature conditions. Accelerated drying can be accom plished by heat-forced air drying or by heating the carrier surface. Drying ovens and infrared heating lamps also can be used to accelerate the removal of the volatile carrier.

A variety of substrates may be used in accordance with the practice of this invention. These substrates include but are not limited to coated and uncoated paper, plastic substrates including films, woven and/or non-woven fabrics of natural and/or synthetic materials. One desirable carrier base is identified as a 70# C2S paper (70# Sterling marketed by Westvaco). Partially coated and uncoated paper stock of both similar and different weights have been found acceptable as a carrier base.

The slurry can be applied to these substrates by various printing or coating techniques, including heatset web offset with and without in-line finishing, gravure, screen printing, pad application, extrusion, and flexographic methods, centrifugal and electrostatic spraying techniques.

The evaporation of the volatile carrier leaves the microcapsules of fragrance deposited and non-adhered to the carrier substrate. The deposited fragrance capsules may be left intact on the paper substrate. Alternatively, a protective overlay may be used to further hold and protect the fragrance capsules until used. An overlay may comprise a laminate material which is removable to expose the fragrance capsules for use. Alternatively, the laminate may comprise the carrier substrate which is folded over on itself and perforated for removal to gain access to the underlying fragrance capsules.

The deposited microcapsules of fragrance carried non-adhered to the paper carrier substrate may be removed by methods such as wiping. The dislodged and free fragrance capsules then are ruptured under desired and controlled pressure conditions to release the fragrance.

The following examples comprise solution mixtures which are usefully employed in preparing the product of the invention:

| Example 1: | | Material | Parts by weight |
|---|---|---|---|
| | 1. | Fragrance capsules at 42% in water .... | 98.3 |
| | 2. | Fumed silica (hydrophilic, Cabot) .... | 1.7 |
| | 3. | Wetting agents at trace amounts less than 0.001% | |
| Example 2: | | | |
| | 1. | Fragrance capsules at 35% in water .... | 97.5 |
| | 2. | Polyethylene oxide (Polyox, Union Carbide) .... | 2.5 |
| | 3. | Wetting agents at trace amounts less than 0.001% | |
| Example 3: | | | |
| | 1. | Fragrance capsules at 55% in water .... | 82.45 |
| | 2. | Iso-propyl alcohol .... | 15.55 |
| | 3. | Fumed silica (hydrophilic, Cabot) .... | 2.00 |
| | 4. | Wetting agents at trace amounts less than 0.001% | |

The product of the invention is useful for dispensing microencapsulated liquids in dry, powder form. The encapsulated liquid is in an essentially non-adhered coating on the substrate, so that it can be readily removed from the substrate. The product has particular utility as a fragrance sampler. It also may be used to dispense cosmetics, skin care products, pharmaceuticals, vitamins and other particulates having therapeutic value for dermal and transdermal use.

The invention has special advantages over prior art products and methods. It permits the choice of normally formulated essential oils, the use of many substrates, and the use of a wide range of binders and inks because less lay - down area is required, so that a higher concentration of fragrance may be dispensed while using less essential oil.

Other uses of the encapsulated particulates, the products containing the particulates and the methods of making the particulates and the products will be apparent to those skilled in the art. Various modifications may be made to the products and methods without departing from the spirit and scope of the invention.


## Claims

1. A product for dispensing a liquid which comprises the liquid contained in microencapsulated dry particulate form and a carrier substrate, wherein the microencapsulated particulates are substantially non-adhered to the carrier substrate.

2. A product as in claim 1, wherein the substrate is selected from the group consisting of coated paper, uncoated paper, plastic films, natural woven fabrics, natural non-woven fabrics, synthetic woven fabrics and synthetic non-woven fabrics.

3. A product as in claim 1 or 2, wherein the microencapsulated particulates are carried in a volatile solvent and deposited on the carrier substrate using printing or coating techniques.

4. A product as in any one of claims 1 to 3, wherein the microencapsulated particulates form a substantially dry powder layer of from about 0.01 millimeter to about 10 millimeters thick on one surface of the carrier substrate.

5. A product as in any one of claims 1 to 4, wherein the microencapsulated liquid comprises a water-insoluble liquid.

6. A method of preparing a microencapsulated liquid product, comprising preparing a slurry by contacting microencapsulated dry particulates with a suspending agent and a wetting agent, applying the slurry so formed to the carrier substrate, and evaporating the wetting agent from the applied slurry, thereby forming a microencapsulated liquid in dry form which is substantially non-adhered to the carrier substrate and which is adapted to be dispensed from the carrier substrate.

7. A method as in claim 6, wherein the suspending agent comprises hydrophilic fumed silicated or polyethylene oxide.

8. A method as in claim 6 or 7, wherein the wetting agent comprises water.

9. A method as in claim 6, 7 or 8, wherein the slurry may be applied to the carrier substrate by printing or coating.

10. A method as in any one of claims 6 to 9, wherein the printing or coating is selected from the group consisting of heatset web offsett with inline finishing, heatset web offset without inline finishing, gravure, screen printing, pad application, extrusion, flexography, electrostatic spraying and contrifugal spraying.

11. A method as in any one of claims 6 to 10, wherein the amount of slurry applied to the substrate comprises an amount which forms a layer of slurry of from about 0.01 millimeter to about 10 millimeters thick on the substrate, advantageously of from about 1.0 millimeter to about 3.0 millimeters thick on the substrate.

12. A method as in any one of claims 6 to 11, wherein the wetting agent is evaporated by increasing the ambient temperature from about 20°C to about 30°C or by heat-forced air drying, or heating the substrate.

13. A method as in any one of claims 6 to 12, wherein the slurry comprises from about 20% to about 45% particulate solids by weight.

14. A method as in any one of claims 6 to 13, wherein the slurry further comprises isopropyl alcohol, a soluble polymer, or a dye solution.

15. A method as in claim 14, wherein said soluble polymer is selected from the group consisting of polyvinyl alcohol, polyvinyl pyrrolidone, hydroxyethyl cellulose, acrylic acid and natural gum.

16. A method of producing a water-insoluble liquid microencapsulated particulate, comprising reacting a precondensate mixture and polymerizing the precondensate mixture so formed under suitable conditions, thereby producting a slurry of water-insoluble liquid microencapsulated particulates in dry powder form.

17. A method as in claim 16, wherein the precondensate mixture comprises melamine/urea, formaldehyde, a cross-linking agent and a water-insoluble liquid material to be microencapsulated.

18. A method, as in claim 16 or 17, wherein the precondensate mixture comprises from about 3% solids content by weight to about 80% solids content by weight.

19. A method as in any one of claims 16 to 18, wherein the water-insoluble liquid material comprises from about 0.1 part by weight for each part solid in the precondensate mixture to about 10 parts by weight for each part solid in the precondensate mixture.

20. A method as in any one of claims 16 to 19, wherein the precondensate mixture is polymerized by adding an acid so that the pH of the precondensate mixture is from about 1.0 to 5.0, preferably from about 1.5 to about 3.5.

21. A method as in any one of claims 16 to 20, wherein the precondensate mixture is agitated for a period of time from about 0.5 hours to about 10 hours, preferably from about 0.75 hours to 8 hours.

22. A method as in any one of claims 16 to 21, wherein the precondensate is heated to a temperature from about 15°C to about 25°C.

23. A method as in any one of claims 16 to 22, wherein the water-insoluble liquid microencapsulated particulates have a diameter from about 5 microns to about 200 microns.

24. A method as in any one of claims 16 to 23, wherein the water-insoluble liquid microencapsulated particulates have a rupture strength from about 5 g/cm to about 500 g/cm.

25. A method as in any one of claims 16 to 24, wherein the precondensate mixture further comprises a dye.

26. A microencapsulated liquid product made by the method of any one of claims 16 to 25.

27. A microencapsulated liquid product made by the method of any one of claims 16 to 25, wherein the product has a rupture strength from about 50 g/cm to about 200 g/cm.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-3 886 084 (A.E. VASSILIADES) * Column 11, lines 43-53; column 12, lines 21-33; column 13, lines 1-7; column 14, lines 45-68 * | 1-3,5,6 ,8,9,12 ,14,16, 17,20, 23 | A 61 K 7/00 |
| X | US-A-3 993 831 (A.E. VASSILIADES) * Column 5, lines 1-35; column 7, lines 41-56; column 8, lines 32-68 * | 1-3,5- 10,14- 18 | |
| X | EP-A-0 247 864 (QMAX TECHNOLOGY GROUP) * Page 2, lines 5-6; page 3, lines 3-13; page 4, lines 53-64; page 5, lines 53-59; page 6, lines 36-41 * | 1-3,5,6 ,9,10, 12,26 | |
| A | US-A-3 516 846 (G.W. MATSON) * Column 5, lines 17-75; column 6, lines 1-39; column 17, lines 13-73 * | 1,2,13, 16,17, 19,20, 21,23 | |

TECHNICAL FIELDS SEARCHED (Int. Cl.5)

A 61 K
B 01 J
B 41 M

The present search report has been drawn up for all claims

| Place of search | Date of completion of the search | Examiner |
|---|---|---|
| THE HAGUE | 06-12-1989 | KERRES P.M.G. |